# FASCICULE DE BREVET EUROPEEN

(11) **EP 0 620 436 B1**
(45) Date de publication et mention de la délivrance du brevet: **25.08.1999**
(21) Numéro de dépôt: 94400640.2
(22) Date de dépôt: 25.03.1994
(51) Int. Cl.: G01N 33/18, G01M 3/04

(54) **Dispositif destiné à détecter des traces de produits polluant dans un milieu aqueux**
Vorrichtung zur Bestimmung von Verunreinigungsspuren in einem wässrigen Medium
Device for detecting traces of pollutants in an aqueous medium

(30) Priorité: 13.04.1993 FR 9304418
(43) Date de publication de la demande: 19.10.1994
(73) Titulaire: INSTITUT FRANCAIS DU PETROLE, 92500 Rueil Malmaison (FR)
(72) Inventeur: Dawans, François, F-78380 Bougival (FR); Huvey, Michel, F-78380 Bougival (FR); Lesage, Jean, F-78990 Elancourt (FR)

(56) Documents cités:
- DE-A- 2 559 233
- DE-A- 3 140 804
- FR-A- 2 254 749
- FR-A- 2 266 157
- FR-A- 2 455 294
- US-A- 3 427 869
- US-A- 3 720 797
- PATENT ABSTRACTS OF JAPAN vol. 8, no. 196 (P-299) (1633) 8 Septembre 1984 & JP-A-59 083 029 (NIPPON DENKI K. K.) 14 Mai 1984

## Description

La présente invention concerne le domaine de la prévention de la pollution d'un milieu aqueux.

Plus particulièrement, la présente invention concerne la détection de certains produits organiques polluants, notamment les hydrocarbures, présents dans un milieu aqueux.

Dans ce domaine, on connaît des systèmes de détection comportant au moins un élément sensible au produit polluant et qui se détériore ou, plus généralement, dont les propriétés mécaniques varient, au contact du produit polluant.

Le principe de fonctionnement, bien connu, de tels systèmes de détection consiste à faire coopérer l'élément sensible avec des moyens capables d'actionner une alarme lorsque l'élément sensible est détérioré par le produit polluant.

Selon les applications envisagées une alarme sonore, visuelle ou tout autre moyen de réaction peut être actionné suite à la dégradation de l'élément sensible.

La dégradation de l'élément sensible peut par exemple déclencher la dispersion d'un produit absorbant ou neutralisant vis-à-vis du polluant.

On a déjà proposé des dispositifs de détection automatiques sensibles à certains produits polluants, notamment les hydrocarbures, comportant deux morceaux d'une bande ou d'un fil assemblés par une colle spéciale qui est détériorée ou dissoute par l'agent polluant. L'assemblage étant maintenu sous tension, l'action de l'agent polluant provoque la rupture de l'assemblage, entraînant le déclenchement d'un signal d'alarme. De tels dispositifs ont, par exemple, été fabriqués par la société canadienne Bennett Pollution Controls Ltd, sous la dénomination "Oil Spill Detection System".

Un tel système présente des difficultés de fabrication et les résultats sont peu fiables, la sensibilité du collage aux agents polluants dépendant énormément de la qualité de fabrication.

On connaît également les dispositifs qui sont basés sur l'utilisation d'un élément sensible massif qui, au contact des produits polluants, est gonflé ou détruit ce qui provoque le déclenchement d'une alarme (cf. le brevet français N° 2 178 950) ; JP-A 083 029.

On connaît encore le brevet français FR 2 266 157 qui décrit une sonde ayant un élément sensible aux hydrocarbures, tubulaire et intercalé entre deux électrodes formés par deux ressorts coaxiaux. L'élément sensible est ici sollicité en cisaillement lorsqu'il gonfle.

Les divers produits proposés jusqu'ici pour constituer l'élément sensible ne donnent pas entièrement satisfaction, car ils s'avèrent trop peu sensibles aux produits polluants (produits selon le brevet français N° 2 178 950) ou, lorsqu'ils sont d'une sensibilité suffisante, ne résistent pas assez longtemps à l'action de la lumière (produits selon le brevet français N° 2 254 749).

Selon un autre concept, décrit dans le document DE 3 140 804, un pipeline est muni d'un système de détection de puits d'hydrocarbure.

Le document DE 3 140 804 décrit un pipeline muni d'un système de détection de fuites d'hydrocarbures. Ce système consiste en un câble plat conducteur, noyé dans un élément isolant sensible aux hydrocarbures, l'ensemble étant enroulé en hélice dans l'épaisseur du pipeline. Aucun élément spécifique de compression n'agit de façon continue sur l'élément sensible.

Afin de pallier certains des inconvénients énoncés ci-dessus, il a été proposé, dans le brevet français FR 2 455 294 déposé au nom de la demanderesse, d'utiliser comme matériau se détériorant au contact des produits, un matériau comportant un copolymère séquencé styrène butadiéne dont une partie au moins est hydrogénée. Préférentiellement, ce matériau comporte des agents stabilisants vis-à-vis des rayons ultra-violets, des agents anti-oxydants et de l'huile 400k dans des proportions déterminées en fonction de l'utilisation envisagée.

Selon cette réalisation l'élément sensible peut être une bande mince maintenue en traction et dont la rupture, par suite de sa détérioration par des hydrocarbures, déclenche un système d'alerte.

Il s'est avéré, à l'usage, qu'un tel détecteur d'hydrocarbures n'est réellement actif qu'en présence de quantités non négligeables d'hydrocarbures dans l'eau. Ce détecteur présente avant tout une fonction de fusible ne se déclenchant qu'en présence de forte quantité d'hydrocarbures.

Pour remédier notamment à ce problème, la présente invention propose un détecteur d'hydrocarbures présents dans un milieu aqueux dont la sensibilité est nettement améliorée vis-à-vis de l'art antérieur, et qui permet donc de détecter des traces d'hydrocarbures dissoutes dans un milieu aqueux.

Un autre avantage de l'invention réside dans la simplicité du dispositif. Un avantage supplémentaire de l'invention réside dans la faible quantité de matériau entrant dans la constitution de l'élément sensible.

Ainsi, la présente invention peut être utilisée pour détecter la pollution de nappes phréatiques.

Une autre utilisation possible de l'invention concerne.la surveillance permanente d'eaux marines fluviales, portuaires, cotières, urbaines, industrielles ou pluviales contre la pollution par des traces d'hydrocarbures.

L'invention peut être utilisée pour la détection d'hydrocarbures oxygénés tels que les phénols, les acides, les éthers, les esters...

La présente invention a pour objet un dispositif destiné à détecter des traces de produits polluants présentes dans un milieu aqueux, comprenant un circuit électrique renfermant au moins un élément sensible présentant la forme d'un film mince réagissant par dégradation à la présence d'hydrocarbures, des moyens électriques coopérant avec ledit élément sensible et un moyen de contrôle de l'état du circuit.

Selon l'invention lesdits moyens électriques sont constitués d'au moins deux contacteurs maintenant en compression ledit élément sensible.

Préférentiellement, l'élément sensible présente une épaisseur comprise entre 0,05 mm et 0,60 mm au niveau de la zone de compression.

Avantageusement, l'élément sensible est constitué d'un élastomère thermoplastique non réticulé par voie chimique.

Plus précisément, l'élément sensible est constitué d'un copolymère multiséquencé styrène-butadiène.

En outre, le dispositif selon l'invention peut comporter un élément de sécurité interrompant le passage du courant électrique (dans le circuit électrique) au cas où le dispositif n'est plus immergé.

Selon un mode de réalisation de l'invention, l'un au moins des contacteurs coopérant avec l'élément sensible présente une indentation au niveau de l'élément sensible.

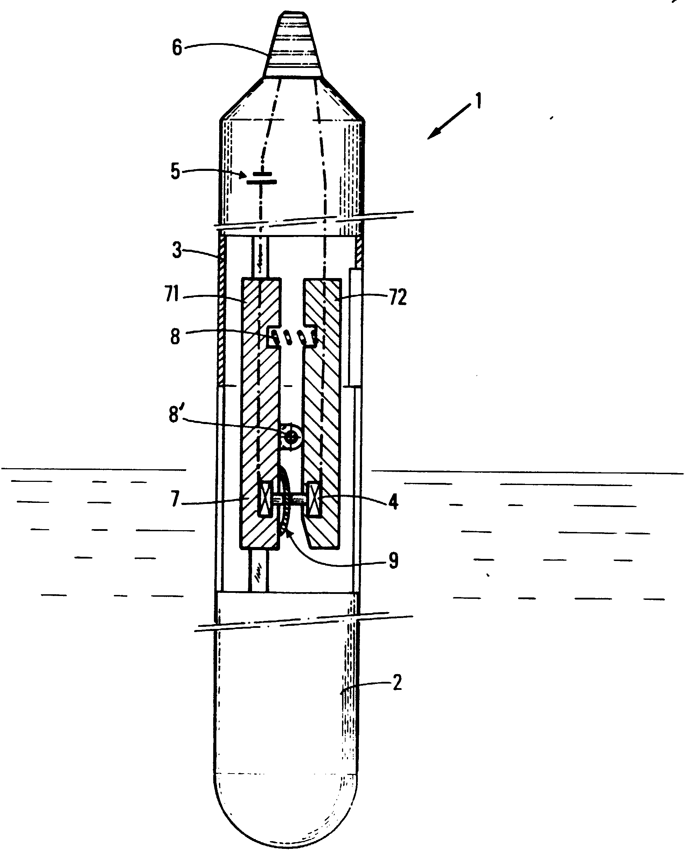

L'invention sera mieux comprise, d'autres éléments et avantages apparaîtront mieux à la lecture de la description qui va suivre, faite à titre illustratif et nullement limitatif, en référence à l'unique figure annexée.

Cette figure représente schématiquement, à titre d'exemple un dispositif 1 détecteur de traces d'hydrocarbures flottant à la surface de l'eau.

Ce dispositif peut-être maintenu en position verticale par un lest 2. Une enveloppe 3 renferme un circuit électrique comprenant essentiellement en série deux contacteurs 4, une alimentation autonome 5 et un moyen de contrôle de l'état de circuit sous forme d'alarme sonore et/ou lumineuse. Un élément supplémentaire de sécurité (non représenté) peut être prévu afin d'éviter d'éventuelles arcs électriques au cas où les contacteurs ne plongeraient plus dans l'eau.

Les contacteurs sont comprimés l'un vers l'autre par tout moyen 7 connu en soi. Selon la figure annexée, ce moyen 7 ressemble à une pince comprenant deux bras 71, 72 dont les extrêmités sont comprimées grâce à un ressort spiralé 8. Ces bras sont réalisés en un matériau isolant et ils comprennent chacun au moins un logement pour un contacteur 4 et des passages pour des fils électriques reliant chaque contacteur aux autres constituants du circuit électrique.

Chaque contacteur 4 présente de préférence une indentation ou zone saillante de forme arrondie ou cylindrique afin d'augmenter la pression sur le matériau, la charge étant alors ponctuelle et non répartie.

Tout moyen capable de régler la force de compression exercée sur l'élément sensible peut avantageusement être prévu.

Un élément sensible 9, sous forme de film mince, d'épaisseur comprise préférentiellement entre 0,05 mm et 0,60 mm est interposé entre les deux contacteurs 4 au niveau de l'indentation lorsque celle-ci est prévue.

La mise en place de l'élément sensible 9 est réalisée par toute technique conventionnelle soit de pose de film mince, soit de revêtement obtenu après évaporation du solvant initialement présent dans un polymère en solution.

L'élément sensible 9 est préférentiellement constitué d'élastomère thermoplastique non réticulé comme par exemple un copolymère multiséquencé styrène-butadiène.

L'élément sensible se dégrade, de façon connue en soi, en présence d'hydrocarbures.

Selon l'invention cette dégradation est favorisée par le fait que l'élément sensible 9 est maintenu en compression entre les deux contacteurs 4.

Lorsque le dispositif selon l'invention est placé dans une eau non polluée, l'élément sensible 9 ne se dégrade pas et le contact ne s'établit donc pas, l'alarme 6 n'est pas actionnée.

Lorsque le dispositif selon l'invention est placé dans de l'eau contenant un hydrocarbure, le matériau sensible 9 flue peu à peu. Le fluage est accéléré par le fait que l'élément sensible 9 est comprimé ; le matériau a ainsi tendance à perdre de sa dureté au fur et à mesure qu'il absorbe des hydrocarbures.

Ainsi après un bref laps de temps (précisé dans les exemples qui vont suivre), et même en présence de faibles traces d'hydrocarbures, l'alarme 6 peut être déclenchée.

Bien entendu, le temps de réponse c'est-à-dire le temps au bout duquel l'alarme se déclenche, varie selon la concentration du milieu aqueux en hydrocarbures, la température du milieu aqueux, la nature du matériau sensible et son épaisseur : les exemples ci-dessous montrent l'influence de ces paramètres.

Il est en outre possible de régler ce temps de réponse par la contrainte de compression exercée au niveau des contacteurs 4, et/ou par la forme et les dimensions de l'indentation des contacteurs.

Les matériaux et les formes des différents constituants du dispositif selon l'invention seront choisis de façon à éliminer tout phénomène d'électrolyse pouvant résulter du passage de courant entre les contacteurs 4 au sein de la phase aqueuse.

Les exemples qui vont suivre mettent notamment en évidence la fiabilité du système selon l'invention, sa sensibilité, l'influence des différents paramètres évoqués ci-avant :

### Exemple 1

Un appareillage est réalisé selon le schéma de la figure annexée.

La source d'alimentation électrique est une pile de 4,5V.

Le signal de détection est un signal lumineux.

Les contacteurs électriques de détection (φ = 3 mm) sont maintenus en compression sous une contrainte de 2MPa, un film de polymère (épaisseur 0,60 mm) étant intercalé entre les contacteurs.

L'appareillage est immergé dans de l'eau pure.

Après trois mois aucun passage de courant n'est décelé entre les deux contacteurs.

### Exemple 2

On renouvelle l'exemple 1 en remplaçant la membrane d'épaisseur 0,60 mm par une membrane d'épaisseur 0,12 mm.

Après trois mois aucun passage de courant n'est décelé.

### Exemple 3

On renouvelle l'exemple 1 en immergeant cette fois le système selon l'invention dans de l'eau saturée de toluène, c'est-à-dire contenant environ 0,05 g de toluène pour 100 g d'eau.

Le temps au bout duquel l'alarme 6 se déclenche est 24 à 48 heures.

### Exemple 4

On renouvelle l'exemple 2 en immergeant le système selon l'invention dans de l'eau saturée de toluène telle que définie dans l'exemple 3.

Le temps de réponse est de 10 à 17 heures.

### Exemple 5

On renouvelle l'exemple 4 en remplaçant la membrane d'épaisseur 0,12 mm par une membrane d'épaisseur 0,08 mm.

Le temps de réponse est de 4 à 5 heures.

### Exemple 6

On renouvelle l'exemple 5, le milieu aqueux étant maintenu à 50° C.

Le temps de réponse est divisé par 2.

Bien entendu diverses modifications et adjonctions pourront être apportées par l'homme de métier au dispositif qui vient d'être décrit sans sortir du cadre de la présente invention.

## Revendications

1. Dispositif destiné à détecter des traces de produits hydrocarbonés polluants présents dans un milieu aqueux, comprenant un circuit électrique renfermant au moins un élément sensible (9) réagissant par dégradation à la présence de traces desdits produits hydrocarbonés polluants, des moyens électriques (4) coopérant avec ledit élément sensible et un moyen de contrôle (6) de l'état dudit circuit caractérisé en ce que lesdits moyens électriques (4) comportent au moins deux contacteurs maintenant en compression ledit élément sensible (9), ledit élément sensible présentant la forme d'un film mince.

2. Dispositif selon la revendication 1 caractérisé en ce que l'élément sensible (9) présente une épaisseur comprise entre 0,05 mm et 0,60 mm au niveau de la zone de compression.

3. Dispositif selon l'une quelconque des revendications 1 ou 2 caractérisé en ce que l'élément sensible (9) est constitué d'un élastomère thermoplastique.

4. Dispositif selon la revendication 3 caractérisé en ce que l'élément sensible (9) est constitué d'un copolymère multiséquencé styrène-butadiène.

5. Dispositif selon la revendication 4 caractérisé en ce que ledit copolymère multiséquencé styrène-butadiène est partiellement ou totalement hydrogéné.

6. Dispositif selon l'une quelconque des revendications précédentes caractérisé en ce qu'il comporte en outre un élément de sécurité interrompant le passage du courant électrique dans le circuit au cas où le dispositif n'est plus immergé.

7. Dispositif selon l'une quelconque des revendications précédentes caractérisé en ce que l'un au moins des contacteurs coopérant avec l'élément sensible présente une indentation au niveau de l'élément sensible.

8. Dispositif selon l'une quelconque des revendications précédentes caractérisé en ce qu'il comporte en outre un moyen de réglage de la force de compression exercée sur l'élément sensible (9).

## Patentansprüche

1. Vorrichtung zur Erfassung der Verunreinigungsspuren durch in einem wässrigen Medium vorhandene kohlenwasserstoffhaltige Produkte, einen elektrischen Kreis umfassend, der wenigstens ein empfindliches Element (9) einschließt, das durch Abbau auf das Vorhandensein von Spuren dieser verunreinigenden kohlenwasserstoffhaltigen Produkte reagiert, weiter elektrische Mittel (4) umfassend, die mit diesem empfindlichen Element zusammenwirken, sowie ein Regelmittel (6) für den Zustand dieses Kreises, dadurch gekennzeichnet, daß diese elektrischen Mittel (4) wenigstens zwei Kontaktschalter umfassen, welche dieses empfindliche Element (9) unter Kompression setzen, wobei dieses empfindliche Element die Form eines dünnen Films aufweist.

2. Vorrichtung nach Anspruch 1, dadurch gekennzeichnet, daß das empfindliche Element (9) eine Dicke zwischen 0,05 mm und 0,60 mm in Höhe der Kompressionszone aufweist.

3. Vorrichtung nach einem der Ansprüche 1 oder 2, dadurch gekennzeichnet, daß das empfindliche Element (9) aus einem thermoplastischen Elastomer gebildet ist.

4. Vorrichtung nach Anspruch 3, dadurch gekennzeichnet, daß das empfindliche Element (9) aus einem Multisequenzcopolymer aus Styrol-butadien gebildet ist.

5. Vorrichtung nach Anspruch 4, dadurch gekennzeichnet, daß dieses Multisequenzcopolymer aus Styrol-butadien teilweise oder vollständig hydriert ist.

6. Vorrichtung nach einem der vorhergehenden Ansprüche, dadurch gekennzeichnet, daß es im übrigen ein Sicherheitselement umfaßt, welches den Stromdurchgang in diesem Kreis für den Fall, daß die Vorrichtung nicht mehr eingetaucht ist, unterbricht.

7. Vorrichtung nach einem der vorhergehenden Ansprüche, dadurch gekennzeichnet, daß wenigstens einer der mit dem empfindlichen Element zusammenwirkenden Kontaktschalter eine Einkerbung in Höhe des empfindlichen Elements hat.

8. Vorrichtung nach einem der vorhergehenden Ansprüche, dadurch gekennzeichnet, daß sie im übrigen ein Mittel zum Regeln der auf das empfindliche Element (9) ausgeübten Kompressionskraft umfaßt.

## Claims

1. A device designed to detect traces of pollutant hydrocarbon products present in an aqueous medium, comprising an electric circuit incorporating at least one sensitive element (9) which reacts by deteriorating in the presence of traces of said pollutant hydrocarbon products, electric means (4) co-operating with said sensitive element and a means for controlling (6) the status of the circuit, characterised in that said electric means (4) include at least two contacts retaining said sensitive element (9) under compression, said sensitive element being the in form of a thin film.

2. A device as claimed in claim 1, characterised in that the sensitive element (9) is of a thickness ranging between 0.05 mm and 0.60 mm on a level with the compression zone.

3. A device as claimed in anyone of claims 1 or 2, characterised in that the sensitive element (9) is made from a thermoplastic elastomer.

4. A device as claimed in claim 3, characterised in that the sensitive element (9) is made from a multi-sequential butadiene-styrene polymer.

5. A device as claimed in claim 4, characterised in that said multi-sequential butadiene-styrene polymer is partially or totally hydrogenated.

6. A device as claimed in any one of the preceding claims, characterised in that it also incorporates a safety element to interrupt the flow of electric current in the circuit if the device is no longer immersed.

7. A device as claimed in any one of the preceding claims, characterised in that at least one of the contacts co-operating with the sensitive element has an indentation on a level with the sensitive element.

8. A device as claimed in any one of the preceding claims, characterised in that it also has a means for regulating the compressive force applied to the sensitive element (9).
